# FASCICULE DE BREVET EUROPEEN

(11) **EP 2 473 627 B1**
(45) Date de publication et mention de la délivrance du brevet: **28.12.2016**
(21) Numéro de dépôt: 10760274.0
(22) Date de dépôt: 02.09.2010
(51) Int. Cl.: C12Q 1/68, A61K 8/72, A61Q 19/00

(54) **SIGNATURE microARN DE LA DIFFÉRENCIATION ÉPIDERMIQUE ET UTILISATIONS**
MICRORNASIGNATUR EPIDERMALER DIFFERENZIATION UND DEREN VERWENDUNGEN
MICRORNA SIGNATURE OF EPIDERMAL DIFFERENTIATION AND USES THEREOF

(30) Priorité: 01.12.2009 FR 0905789; 02.09.2009 US 239154 P
(43) Date de publication de la demande: 11.07.2012
(73) Titulaire: L'Oréal, 75008 Paris (FR)
(72) Inventeur: MARIONNET, Claire, F-92410 Ville D'Avray (FR); BERNERD, Françoise, F-06100 Nice (FR)
(74) Mandataire: Almond-Martin, Carol
(86) Numéro de dépôt international: PCT/EP2010/062891
(87) Numéro de publication internationale: WO 2011/026909

(56) Documents cités:
- WO-A1-2009/018493
- WO-A1-2009/036332
- WO-A1-2009/148137
- WO-A2-2008/142567
- WO-A2-2009/043353
- WO-A2-2010/018585
- LENA A M ET AL: "miR-203 represses 'stemness' by repressing DeltaNp63.", CELL DEATH AND DIFFERENTIATION JUL 2008 LNKD- PUBMED:18483491, vol. 15, no. 7, juillet 2008 (2008-07), pages 1187-1195, XP002593224, ISSN: 1350-9047
- MAZAR JOSEPH ET AL: "Protein-coding and non-coding gene expression analysis in differentiating human keratinocytes using a three-dimensional epidermal equivalent.", MOLECULAR GENETICS AND GENOMICS : MGG JUL 2010 LNKD- PUBMED:20499100, vol. 284, no. 1, 25 mai 2010 (2010-05-25), pages 1-9, XP002593226, ISSN: 1617-4623
- SONKOLY ENIKÖ ET AL: "Protein kinase C-dependent upregulation of miR-203 induces the differentiation of human keratinocytes.", THE JOURNAL OF INVESTIGATIVE DERMATOLOGY JAN 2010 LNKD- PUBMED:19759552, vol. 130, no. 1, 17 septembre 2009 (2009-09-17), pages 124-134, XP002593223, ISSN: 1523-1747
- WENGUANG ZHANG ET AL: "A subset of skin-expressed microRNAs with possible roles in goat and sheep hair growth based on expression profiling of mammalian microRNAs", OMICS A JOURNAL OF INTEGRATIVE BIOLOGY, MARY ANN LIEBERT, NEW YORK, NY, US LNKD- DOI:10.1089/OMI.2006.0031, vol. 11, no. 4, 1 janvier 2007 (2007-01-01), pages 385-396, XP009110632, ISSN: 1536-2310
- LIN SHI-LUNG ET AL: "CHAPTER 4: Recent Application of Intronic MicroRNA Agents in Cosmetics", 1 janvier 2008 (2008-01-01), CURRENT PERSPECTIVES IN MICRORNAS (MIRNA), SPRINGER NETHERLANDS LNKD- DOI:10.1007/978-1-4020-8533-8_4, PAGE(S) 51 - 72, XP009136584, ISBN: 978-1-4020-8532-1 * abrégé pages 66-68 figure 4.7
- ABERDAM D ET AL: "miRNAs, 'stemness' and skin", TRENDS IN BIOCHEMICAL SCIENCES, ELSEVIER, HAYWARDS, GB LNKD- DOI:10.1016/J.TIBS.2008.09.002, vol. 33, no. 12, 1 décembre 2008 (2008-12-01), pages 583-591, XP025691283, ISSN: 0968-0004 [extrait le 2008-11-25]
- HARDING CLIVE R: "The stratum corneum: structure and function in health and disease.", DERMATOLOGIC THERAPY 2004 LNKD- PUBMED:14728694, vol. 17 Suppl 1, 2004, pages 6-15, XP002593225, ISSN: 1396-0296

## Description

La présente invention est dans le domaine cosmétique, elle est relative à la peau. Elle s'inscrit plus généralement dans le cadre de la caractérisation de l'état de différenciation épidermique de la peau et du traitement de troubles, d'affections ou pathologies liées à la différenciation épidermique.

L'épiderme est un épithélium pluristratifié qui exerce une fonction barrière de protection contre son environnement et ses agressions. Il est composé majoritairement de kératinocytes, qui prolifèrent dans la couche la plus profonde de l'épiderme puis qui entrent en différenciation en migrant de la profondeur vers la superficie de l'épiderme. En surface de l'épiderme, les kératinocytes les plus différenciés, appelés cornéocytes constituent la couche cornée, hautement protectrice. L'épiderme est donc à la fois le siège d'une multiplication cellulaire, qui a lieu au sein de la couche basale, et également le siège d'une différenciation cellulaire, au sein de la couche intermédiaire, qui conduit à la formation d'une structure protectrice bien différenciée, la couche cornée. Le cycle complet dure une vingtaine de jours. Ce programme correspond à l'expression de nombreux gènes spécifiques successivement activés puis réprimés de manière séquentielle et coordonnée. Ce programme implique un grand nombre de facteurs de transcription, enzymes, lipides et protéines structurales. De nombreux facteurs de croissance, le calcium, les dérivés des vitamines A et D3 sont d'importants régulateurs de la prolifération et de la différenciation kératinocytaires.

Certains états physiologiques ou pathologiques, de même certaines agressions de la surface de la peau ou via la circulation sanguine, conduisent à une dérégulation de ce processus de différenciation épidermique coordonné dans le temps et dans l'espace.

Dans les pathologies cutanées associées à des modifications du programme de différenciation épidermique, on notera de nombreuses maladies, génétiques ou non [14-15], comme le psoriasis, la dermatite atopique, l'acné, les ichthyoses, le syndrome de Netherton, le pityriasis rubra pilaire, la kératose pilaire, la maladie de Darier, les kératodermies palmoplantaires et les porokératoses.

Dans les circonstances plus physiologiques associées à des modifications de la différenciation épidermique, on citera par exemple : l'âge avec des anomalies de la différenciation terminale et une diminution de la fonction barrière [17], la cicatrisation qui se caractérise par une phase de modification de l'homéostasie épidermique (hyperprolifération, migration, ré-épithélialisation et restauration de la différenciation terminale), les xéroses hivernales ou liées à l'âge (peau sèche, avec processus d'accumulation de squames) [18].

Dans les perturbations dues à des acteurs extérieurs induisant des désordres du processus de différenciation épidermique, on mentionnera par exemple [19]: les expositions aux UV solaires, à la chaleur, à des agents exfoliants, des agents desquamants, des agents délipidants, ou des agressions mécaniques.

Dans les cas des affections légères, des émollients et des kératolytiques sont préconisés. Ces traitements n'agissent pas sur la balance prolifération/différenciation, mais ont pour but de rendre les lésions tolérables pour le malade et ils n'ont souvent qu'un effet suspensif.

Pour les affections plus sévères, ce sont l'acide rétinoïque et ses dérivés, la vitamine D3, susceptibles de réguler la prolifération et la différenciation des kératinocytes, qui sont utilisés depuis plusieurs années. Dans certains cas, le méthotrexate et la cyclosporine sont également préconisés. Tous ces traitements ont des effets secondaires importants, très lourds pour les patients.

Afin de caractériser au mieux l'état de différenciation épidermique d'une peau ou d'un épithélium de culture de type « peau reconstruite », et de pouvoir évaluer des molécules pouvant moduler le processus de différenciation épidermique, les outils techniques disponibles à ce jour sont les suivants :
- Analyse morphologique et marqueurs biochimiques des stades de différenciation épidermique (immunohistochimie). C'est une technique qui ne permet d'analyser, que soit très globalement (histologie topographique), soit marqueur par marqueur (immunohistochimie), les acteurs protéiques connus de la différenciation. Elle ne permet pas d'avoir une signature de l'état de la peau. Elle est lourde et nécessite de disposer de certains outils comme des anticorps qui soient spécifiques de marqueurs recherchés, ce qui n'est pas toujours le cas. D'autre part, c'est une technique difficilement quantitative.
- Analyse transcriptomique : c'est une méthode très utilisée pour obtenir des signatures moléculaires d'un tissu sain ou pathologique. Cette technique est basée sur l'analyse de l'expression des transcrits ARNm d'un échantillon. Elle peut être très globale, avec l'utilisation des puces à ADN qui peuvent contenir jusqu'à 12 000 gènes. Cependant elle présente certains inconvénients, notamment elle nécessite des appareillages coûteux (type système Affymétrix^{™}) mais surtout génère une quantité de données très importante souvent difficilement exploitables qu'il faut prendre en charge par un service de bioinformatique performant.
- Analyse protéomique : elle permet de faire un état des lieux global de l'ensemble des protéines présentes dans un tissu. Elle utilise encore beaucoup les techniques de gels d'électrophorèse bi-dimensionnelle et est donc très lourde à mettre en oeuvre. De plus l'identification précise des protéines nécessite des étapes supplémentaires et des appareils très coûteux et requérant une expertise particulière.

Il existe donc un besoin d'une méthode permettant facilement et rapidement de caractériser l'état de différenciation épidermique d'une peau ou d'un épithélium de culture.

Les présents inventeurs ont mis en évidence, de manière parfaitement inattendue, l'existence d'une **signature microARN** de la différenciation épidermique, permettant non seulement de caractériser l'état de différenciation épidermique, mais également d'envisager divers traitements des troubles liés au dérèglement de cet état de différenciation.

Les microARNs (miRNAs) découverts depuis 1993 sont des petits **ARNs endogènes** qui sont impliqués dans l'interférence par ARN : ils peuvent cibler des ARN messagers (ARNm) et générer leur dégradation ou empêcher leur traduction. Ces miRNAs jouent donc des **rôles de régulation très importants** dans la cellule. De plus, ils constituent une des classes de molécules régulatrices les plus abondantes. Ils ont une origine endogène, issus de pri-miRNA codés par le génome. A ce jour environ 700 microARN humains ont été identifiés. Leurs fonctions et leurs cibles n'ont pas toutes été élucidées ou démontrées.

Ils jouent un rôle crucial dans la régulation de diverses voies cellulaires et peuvent être impliqués dans des pathologies humaines. Les miRNAs ont un rôle primordial dans la régulation du développement, de la différenciation, de la prolifération et de l'apoptose (pour revues : [1] et [3]). Par exemple chez l'homme, miR-17-5p et miR-20 sont des régulateurs de la prolifération [4], miR-223 est impliqué dans la granulopoïèse [5].

La dérégulation de l'expression des miRNA peut contribuer à des pathologies humaines. Certains miRNAs fonctionnent comme des suppresseurs de tumeurs ou d'autres comme des oncogènes. A l'heure actuelle, il existe très peu d'études sur les microARN dans la peau, et tout particulièrement dans la peau humaine.

Dans la peau animale, les microARNs exprimés ont été clonés, d'abord chez la souris. Ils ont un rôle important dans la morphogénèse de l'épiderme et du poil [8]. Plus récemment l'implication des microARNs dans la croissance du poil a été montrée chez la chèvre et le mouton [10]. Très récemment, dans la peau de souris, le microARN miR203 a été identifié comme un acteur important dans l'induction de la différenciation épidermique, en réduisant le potentiel prolifératif cellulaire [11].

Chez l'homme, l'expression des microARNs a été étudiée dans la peau normale et comparée à celle de la peau psoriasique et de la peau d'eczéma. Le microARN miR203 est très exprimé dans la peau (comparé à d'autres organes) et uniquement par les kératinocytes. Il a été identifié comme l'un des microARNs surexprimé dans la peau psoriasique ([12] et WO2008/142567) et les auteurs de WO2008/142567 ont exposé des résultats suggérant une différence d'expression de miR203 entre la couche basale et les couches suprasales de l'épiderme, sans toutefois suggérer un quelconque lien avec l'état de différenciation épidermique desdits kératinocytes.

Enfin, il a été montré chez l'homme que certains microARNs (miR -221 et miR-222) étaient impliqués dans le contrôle de la progression du mélanome [13].

Dans la mesure où, pour chaque microARN, il existe plusieurs dizaines de gènes cibles, les présents inventeurs proposent :
- d'utiliser les microARNs comme marqueurs de la différenciation. Ils permettent de part leur nombre beaucoup moins important que les ARN messagers de réaliser des profils microARN d'une situation, d'un tissu, de manière plus simple, plus rapide et moins coûteuse que les méthodes classiques globales de transcriptomique ;
- d'utiliser la signature moléculaire microARN de la différenciation épidermique pour identifier des modulateurs en vue de traitements des affections bénignes ou plus graves associées à des désordres de la différenciation épidermique. Par modulateurs il faut entendre des entités chimiques ou issues d'extraits naturels, des formulations complexes, des siRNA, des inhibiteurs de miRNAs tels que des antagomirs, mais aussi des systèmes instrumentaux utilisant la lumière, des effets mécaniques sur la peau, des injections ;
- d'utiliser les miRNAs ou des modulateurs de ces miRNAs en vue de traitements cosmétiques ou thérapeutiques dans les affections de la différenciation ou de la prolifération épidermique ;
- d'utiliser cette signature miRNAs pour évaluer l'efficacité d'un traitement cosmétique ou thérapeutique de la différenciation épidermique ;
- d'utiliser la signature moléculaire microARN de la différenciation épidermique pour effectuer un diagnostic d'un épiderme normal ou pathologique ;

A cet effet, il est proposé d'utiliser le dosage de l'expression de certains microARNs identifiés.

En effet, de manière étonnante, par l'analyse systématique de l'expression des microARNs dans des kératinocytes épidermiques, les présents inventeurs ont montré que lorsque le processus de différenciation épidermique était engagé et aboutissait à la formation d'une couche cornée, les microARNs humains *miR-141, miR-148a, miR-182, miR-224, miR-26a, miR-26b, miR-361-5p, miR-425, miR-455-3p, miR-92b* étaient surexprimés dans des kératinocytes d'épiderme différencié par rapport à des kératinocytes non différenciés prolifératifs, n'exprimant pas de marqueurs de différenciation épidermique connus et ne formant pas de cornéocytes. Ces dix microARNs représentent donc une signature moléculaire de la différenciation kératinocytaire.

A l'inverse, dans des kératinocytes très indifférenciés et prolifératifs, les microARNs humains *let-7i, miR-22, miR-221, miR-222, miR-29a, miR-29b, miR-663, miR-30a, miR-30c* sont surexprimés dans les kératinocytes non différenciés par rapport à l'épiderme différencié. Ces neuf microARNs représentent à l'inverse un état d'indifférenciation épidermique, associé à une prolifération intense.

La présente invention concerne donc cette signature microARN de la différenciation épidermique et diverses utilisations de cette signature, notamment pour évaluer ou qualifier un état de différenciation épidermique, comme nouvel outil de diagnostic de désordre de la différenciation physiologique ou pathologique, comme cible pour corriger un défaut de différenciation épidermique ou comme biomarqueur et outil de screening pour tester des agents modulateurs de la différenciation épidermique. La présente description divulgue également l'utilisation de microARNs de cette signature, ou de modulateurs de ces microARNs, dans des applications thérapeutiques et cosmétiques, notamment sous forme de compositions.

Dans le cadre de la présente invention, les termes ci-dessous ont plus particulièrement la signification suivante :
**MicroARN** (ou micro ARN ou miRNA) : Les microARNs sont des ARN simple-brin, longs d'environ 20 à 25 nucléotides, plus généralement de 21 à 24 nucléotides. Les miRNAs sont des répresseurs qui agissent après la transcription d'un gène en ARNm : en effet, en s'appariant à des ARN messagers, ils guident leur dégradation, ou la répression de leur traduction en protéine.

La production de miRNAs est sous le contrôle étroit d'une régulation transcriptionnelle et post-transcriptionnelle. Les gènes de miRNA sont transcrits par l'ARN polymérase Il sous la forme de longs transcrits primaires ou précurseurs appelés « pri-miRNA ». Les miRNAs précurseurs sont clivés par voie enzymatique dans le noyau de la cellule par une ARNase III de classe 2 (Drosha), pour former les « pre-miRNA ». Le « pre-miRNA » est un ARN long d'environ 70 nucléotides, replié en tige-boucle imparfaite par complémentarité de bases entre la première moitié et la deuxième moitié de sa séquence. Les pre-miRNAs sont ensuite exportés vers le cytoplasme où ils se lient à une autre nucléase (Dicer) et au complexe RISC (RNA-induced silencing complex) qui contient les protéines TRBP (transactivation-responsive RNA binding protein) et Ago2 (Argonaute 2). Du fait de la liaison, la protéine Ago2 clive les extrémités 3' du précurseur miRNA, générant ainsi le duplex miRNA mature. Seul le brin spécifique de l'ARNm cible du miRNA est gardé (réaction thermodynamique) au sein du complexe, l'autre brin est enlevé et dégradé. L'ARNm cible est alors chargé au sein du complexe RISC et dégradé.

Bien que la répression traductionnelle soit la voie d'extinction (« silencing ») la plus observée dans les cellules animales, de récentes études montrent que les miRNAs peuvent également affecter le taux d'ARNm cible.

**Hsa-miRxx** : il s'agit de la dénomination des mircro ARN identifiés chez l'homme (hsa signifiant homo sapiens). Les séquences de tous les microARNs connus sont répertoriées dans des bases telles que miRBase ou microRNAdb, où ils sont identifiés de manière unique par leur numéro d'accession (xx). Dans ce qui suit, le numéro hsa-miRxx est utilisé pour faire référence à la séquence miRNA mature.

En particulier, les microARNs ci-dessous ont la séquence suivante :
**hsa-miR-141** : miRNA mature : UAACACUGUCUGGUAAAGAUGG (SEQ ID N°1 )
**hsa-miR-148a** : miRNA mature : UCAGUGCACUACAGAACUUUGU (SEQ ID N°2)
**hsa-miR-182** : miRNA mature : UUUGGCAAUGGUAGAACUCACACU (SEQ ID N°3)
**hsa-miR-224** : miRNA mature : CAAGUCACUAGUGGUUCCGUU (SEQ ID N°4)
**hsa-miR-26a (hsa-miR-26a-1 et hsa-miR-26a-2):** miRNA mature : UUCAAGUAAUCCAGGAUAGGCU (SEQ ID N°5)
**hsa-miR-26b** : miRNA mature : UUCAAGUAAUUCAGGAUAGGU (SEQ ID N°6)
**hsa-miR-361-5p** : miRNA mature : UUAUCAGAAUCUCCAGGGGUAC (SEQ ID N°7)
**hsa-miR-425** : miRNA mature : AAUGACACGAUCACUCCCGUUGA (SEQ ID N°8)
**hsa-miR-455-3p** : miRNA mature : GCAGUCCAUGGGCAUAUACAC (SEQ ID N°9)
**hsa-miR-92b** : miRNA mature : UAUUGCACUCGUCCCGGCCUCC (SEQ ID N°10)
**hsa-let-7i** : miRNA mature : UGAGGUAGUAGUUUGUGCUGUU (SEQ ID N°11)
**hsa-miR-22:** miRNA mature : AAGCUGCCAGUUGAAGAACUGU (SEQ ID N°12)
**hsa-miR-221:** miRNA mature : AGCUACAUUGUCUGCUGGGUUUC (SEQ ID N°13)
**hsa-miR-222** : miRNA mature : AGCUACAUCUGGCUACUGGGU (SEQ ID N°14)
**hsa-miR-29a** : miRNA mature : UAGCACCAUCUGAAAUCGGUUA (SEQ ID N°15)
**hsa-miR-29b** : miRNA mature : UAGCACCAUUUGAAAUCAGUGUU (SEQ ID N°16)
**hsa-miR-663** : miRNA mature : AGGCGGGGCGCCGCGGGACCGC (SEQ ID N°17)
**hsa-miR-30a** : miRNA mature : UGUAAACAUCCUCGACUGGAAG (SEQ ID N°18)
**hsa-miR-30c** : miRNA mature : UGUAAACAUCCUACACUCUCAGC (SEQ ID N°19).

Le suffixe 'hsa' a parfois été omis dans le cadre de cette description, il s'agit toutefois de désigner ces mêmes 19 microARNs, décrits ci-dessus.

**Différenciation épidermique** (kératinisation ou cornification) : phénomène au sein de l'épiderme assurant le renouvellement continuel de la peau humaine. La différenciation épidermique regroupe l'ensemble des phénomènes moléculaires, biochimiques et morphologiques qui assurent la transformation de la cellule souche de l'épiderme en une cellule cornée anucléée ou cornéocyte, par le biais de différents stades de différenciation épidermique.

Cette différenciation terminale permet la constitution de structures spécialisées comme la couche cornée assurant à l'organisme une barrière efficace contre l'environnement. Au cours de la différenciation épidermique, les kératinocytes de la couche la plus profonde de l'épiderme se divisent pour donner naissance à deux cellules-filles identiques, dont l'une reste sur place alors que la seconde migre vers la couche supérieure, la couche de différenciation, en subissant des modifications morphologiques et biochimiques.

**kératinocytes non différenciés** (ou indiffenciés ou undifférenciés), ou encore kératinocytes basaux (ou de la couche basale, ou de l'assise basale) : kératinocytes exprimant les kératines K5 et K14, et ayant une activité mitotique.

**kératinocytes différenciés :** kératinocytes n'ayant plus d'activité mitotique et ayant commencé la différenciation terminale, notamment par l'induction des kératines K1 et K10.

**Antagomir :** il s'agit d'une nouvelle classe d'oligonucléotides synthétisés chimiquement. Ils sont utilisés pour éteindre (« silencing ») l'action des microARNs endogènes. Un antagomir est un petit ARN synthétique qui est parfaitement complémentaire à un microARN ciblé, avec une ou des modification(s) de base pour inhiber le clivage par Ago2 (d'autres modifications sont également souvent introduites en plus pour que l'antagomir soit plus résistant à la dégradation).

Les antagomirs sont utilisés de manière fréquente pour inhiber de façon constitutive l'activité de microARNs spécifiques. De très nombreux antagomirs ont été publiés. Tous les miRNAs sont susceptibles d'être inhibés spécifiquement par un antagomir.

Seule l'information de la séquence du microARN ciblé est nécessaire pour l'élaboration d'un antagomir capable d'inhiber l'activité dudit microARN ciblé.

D'autres inhibiteurs de miRNAs sont par exemple des miRCURY LNA™ microRNA Knockdown Probes (Exiqon) ou des miScript miRNA inhibitors de Qiagen.

La présente invention concerne une signature microARN de la différenciation épidermique, et donc une signature représentative de la balance entre prolifération et différenciation.

Selon un **premier** aspect, la présente invention concerne plus spécifiquement une méthode de détermination de l'état de différenciation épidermique d'un kératinocyte humain au sein d'un échantillon d'épithélium humain. Une telle méthode comprend une étape de détermination du niveau d'expression au sein dudit kératinocyte, des microARNs suivants identifiés par les inventeurs, à savoir hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c. Ces 19 miRNAs sont appelés microARNs, ou miRNAs de l'invention.

Le niveau d'expression des microARNs est comparé au niveau d'expression moyen desdits microARNs dans des kératinocytes non différenciés, de préférence des kératinocytes en culture non différenciés, ou bien dans des kératinocytes épidermiques différenciés.

Les méthodes de détection et de quantification des miRNAs sont multiples et bien connues de l'homme du métier. L'exemple 3 détaille les techniques les plus utilisées.

Par niveau moyen d'expression d'un microARN au sein de kératinocytes non différenciés, notamment de kératinocytes en culture non différenciés, ou au sein de kératinocytes épidermiques différenciés, on entend le niveau d'expression généralement observé dans ce type de cellules, de préférence correspondant à la moyenne du niveau d'expression déterminé au sein d'au moins 5 kératinocytes différents, représentatifs de la population de kératinocytes en culture non différenciés, ou bien représentatifs de la population de kératinocytes épidermiques différenciés. De préférence, il s'agit de la moyenne d'au moins 10 mesures, voire 20 ou 100 mesures différentes.

De préférence, les kératinocytes en culture non différenciés, ou bien les kératinocytes épidermiques différenciés dont il est question sont des kératinocytes provenant de la même souche ou de la même population que le kératinocyte sous étude.

Dans le cadre de la mise en oeuvre de la méthode telle que mentionnée plus haut, le kératinocyte sous étude est considéré comme différencié ou en cours de différenciation, si le niveau d'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, est statistiquement supérieur au niveau d'expression moyen desdits microARNs dans des kératinocytes non différenciés, par exemple des kératinocytes en culture non différenciés.

Par niveau statistiquement supérieur, on entend que la différence observée est significative d'un point de vue statistique, notamment qu'elle est supérieure à l'écart-type. Une valeur est dite statistiquement supérieure à une autre si la différence entre les deux valeurs est plus importante que l'incertitude existant sur les mesures.

De préférence, le niveau supérieur est au moins supérieur de 20% au niveau moyen, de préférence, il est au moins supérieur de 50%. Selon des mises en oeuvre particulièrement préférées, le niveau d'expression des microARNs est au moins égal à une fois et demi le niveau moyen desdites microARNs, de préférence il est supérieur ou égal à deux fois le niveau moyen des microARNs choisis.

De préférence, le niveau d'expression d'au moins l'un des microARNs choisi parmi hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, est statistiquement supérieur à tous les niveaux d'expression dudit microARN dans tous les kératinocytes en culture non différenciés ayant permis d'obtenir le niveau moyen d'expression dudit microARN.

Alternativement, la description divulgue qu'on pourra également conclure que le kératinocyte sous étude est différencié ou en cours de différenciation, si le niveau d'expression des microARNs hsa-let-7i, hsa-miR-22, hsa-miR-221, hsa-miR-222, hsa-miR-29a, hsa-miR-29b, hsa-miR-663, hsa-miR-30a et hsa-miR-30c, est sensiblement identique au niveau d'expression desdits microARNs dans des kératinocytes épidermiques différenciés. De préférence, c'est le cas d'au moins 2, voire 3, 5, 8 ou l'intégralité des microARNs choisis parmi hsa-let-7i, hsa-miR-22, hsa-miR-221, hsa-miR-222, hsa-miR-29a, hsa-miR-29b, hsa-miR-663, hsa-miR-30a et hsa-miR-30c.

Par niveau sensiblement identique, on entend que la différence de niveau observée n'est statistiquement pas significative, par exemple elle est inférieure à l'incertitude existant sur la mesure de niveau d'expression dudit microARN.

A l'inverse, dans le cadre de la mise en oeuvre de la méthode de l'invention, il sera considéré que le kératinocyte sous étude est un kératinocyte dans un état non différencié ou peu différencié, si le niveau d'expression des microARNs hsa-let-7i, hsa-miR-22, hsa-miR-221, hsa-miR-222, hsa-miR-29a, hsa-miR-29b, hsa-miR-663, hsa-miR-30a et hsa-miR-30c, est statistiquement supérieur au niveau d'expression moyen desdits microARNs dans des kératinocytes épidermiques différenciés.

Alternativement, la description divulgue qu'on pourra également conclure que le kératinocyte sous étude est dans un état non différencié, aussi dit indifférencié ou undifférencié, ou dans un état peu différencié, si le niveau d'expression d'au moins l'un des microARNs choisi parmi hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, est sensiblement identique au niveau d'expression dudit microARN dans des kératinocytes épidermiques non différenciés. De préférence, c'est le cas d'au moins 2, voire 3, 5, 8 ou l'intégralité des microARNs choisis parmi hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b.

La présente invention concerne également une méthode d'évaluation du différentiel de différenciation épidermique existant au sein d'un échantillon d'épithélium humain. Par cette méthode, il est possible de caractériser l'état de différenciation de cet échantillon, et ainsi par exemple de déterminer s'il est conforme à l'état de différenciation normal d'un épithélium humain sain, ou bien caractéristique d'une pathologie.

Selon cette méthode de l'invention, il est possible de déterminer le différentiel de différenciation épidermique existant entre deux couches de kératinocytes de cet épithélium, c'est-à-dire la différence existant entre ces deux couches en terme de différenciation épidermique, et donc de définir si une couche est dans un état plus différencié qu'une autre couche de cet épithélium, ou bien à l'inverse dans un état plus prolifératif.

La méthode d'évaluation du différentiel de différenciation épidermique selon l'invention comprend une étape de comparaison, entre deux couches de kératinocytes dudit épithélium, du niveau d'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c.

En effet, comme détaillé plus haut, les inventeurs ont mis en évidence les différences importantes de degré d'expression qu'il existe au niveau de ces 19 microARNs, dits microARNs de l'invention, au sein de kératinocytes fortement différenciés, et au sein de kératinocytes non différenciés. L'étude des différences de niveau d'expression de ces microARNs permet donc de conclure à l'existence d'une différence entre l'état de différenciation épidermique d'une couche, par rapport à l'autre couche de kératinocytes de l'épithélium sous étude.

Bien qu'il soit possible d'établir une différence de niveau d'expression de plusieurs microARNs entre les deux couches, la description divulgue que le différentiel de différenciation épidermique est mis en évidence dès lors qu'il existe un niveau d'expression significativement différent dans une couche par rapport à l'autre, pour au moins un des microARNs de l'invention.

Selon cette méthode, le différentiel de différenciation épidermique entre les deux couches se caractérise notamment par une expression plus importante des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b dans une couche par rapport à l'autre. La différence d'expression desdits microARNs doit être d'un ordre de grandeur tel que cette différence est significative d'un point de vue statistique.

De préférence, la différence de niveau d'expression des microARNs est d'un facteur égal ou supérieur à 1,3, de préférence supérieur à 1,5, voire même égal ou supérieur à un facteur 2.

Selon une mise en oeuvre préférée de l'invention, le différentiel de différenciation épidermique entre les deux couches de l'épithélium se caractérise par une expression plus importante des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, dans une couche par rapport à l'autre. La différence de niveau d'expression des microARNs dans une couche par rapport à l'autre n'est pas nécessairement d'un facteur identique pour tous les microARNs de la liste.

Selon cette méthode, le différentiel de différenciation épidermique entre les deux couches peut également se caractériser par une expression plus importante des microARNs hsa-let-7i, hsa-miR-22, hsa-miR-221, hsa-miR-222, hsa-miR-29a, hsa-miR-29b, hsa-miR-663, hsa-miR-30a et hsa-miR-30c dans une couche par rapport à l'autre. Une nouvelle fois, la différence de niveau d'expression à laquelle il est fait référence s'entend d'une différence significative d'un point de vue statistique.

De préférence, la différence de niveau d'expression des microARNs est d'un facteur égal ou supérieur à 1,3, de préférence supérieur à 1,5, voir même égal ou supérieur à un facteur 2.

Selon une mise en oeuvre préférée de l'invention, le différentiel de différenciation épidermique entre les deux couches de l'épithélium se caractérise par une expression plus importante des microARNs hsa-let-7i, hsa-miR-22, hsa-miR-221, hsa-miR-222, hsa-miR-29a, hsa-miR-29b, hsa-miR-663, hsa-miR-30a et hsa-miR-30c, dans une couche par rapport à l'autre. La différence de niveau d'expression des microARNs dans une couche par rapport à l'autre n'est pas nécessairement d'un facteur identique pour tous les microARNs de la liste.

Dans le cadre de la présente invention, l'échantillon d'épithélium, de peau ou d'épiderme dont il est question peut être de différentes sources, il peut s'agir aussi bien d'un épithélium de culture, par exemple un épithélium reconstitué, type « peau reconstruite » ou bien d'un échantillon obtenu d'un individu, par exemple à partir d'une biopsie. Ledit échantillon peut donc provenir d'une peau humaine.

La comparaison du niveau d'expression des microARNs de l'invention peut être réalisée entre deux couches quelconques de l'échantillon d'épithélium sous étude. S'il s'agit d'un échantillon de peau humaine, la comparaison est de préférence opérée entre deux couches de kératinocytes opposées, par exemple entre les kératinocytes de la couche basale et ceux de la couche cornée de l'épithélium. Il est à noter à ce propos que des études récentes indiquent en effet que les cornéocytes produisent ou contiennent des microARNs.

La comparaison du niveau d'expression peut également être réalisée entre deux couches assez proches de l'échantillon, par exemple afin de mettre en évidence les différents stades de différenciation.

La présente invention concerne également diverses applications des méthodes de caractérisation décrites ci-dessus, et donc diverses utilisations de la signature microARNs mise en évidence par les inventeurs, notamment comme outil de diagnostic de désordres de la différenciation, physiologiques ou pathologiques, comme cible pour corriger un défaut de différenciation épidermique ou comme biomarqueur et outil de screening pour tester des agents modulateurs de la différenciation épidermique.

Selon un **second** aspect, l'invention concerne donc un procédé de détermination de l'efficacité d'un traitement effectué sur un épithélium. Un tel procédé comprend la comparaison, avant et après traitement, de la signature microARN de la différenciation épidermique de l'épithélium. Le procédé comprend donc la comparaison du niveau d'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c au sein de kératinocytes de cet épithélium, avant et après traitement, ledit procédé n'étant pas mis en oeuvre sur des êtres humains.

En effet, les inventeurs ont mis en évidence la variation du niveau d'expression de ces 19 microARNs au cours du processus de différenciation épidermique se déroulant dans l'épiderme. La modification du niveau d'expression de ces microARNs est donc représentative d'une modification de l'état de différenciation en réponse au traitement testé. Une nouvelle fois, la modification du niveau d'expression s'entend d'une modification significative d'un point de vue statistique, donc d'intensité suffisante et reproductible, observable sur un nombre conséquent de kératinocytes représentatifs de l'épithélium sous étude.

Alternativement, le procédé de détermination de l'efficacité d'un traitement appliqué sur un épithélium au sens de la présente invention peut également comprendre la comparaison, avant et après le traitement testé, du différentiel de différenciation épidermique de cet épithélium. Selon cette variante du procédé, on détermine donc, avant traitement, le différentiel de différenciation épidermique existant entre deux couches de l'épithélium et on compare le résultat obtenu au différentiel de différenciation épidermique existant entre ces deux mêmes couches après traitement. Il est ainsi déterminé si le traitement testé a modifié ou non le différentiel existant entre ces deux couches, par exemple en réduisant la différence d'état de différenciation entre les deux couches, c'est-à-dire en réduisant les différences de niveaux d'expression des microARNs de l'invention entre les deux couches, ou bien en augmentant le différentiel de différenciation épidermique. Ledit procédé n'est pas mis en oeuvre sur des êtres humains.

Le traitement testé par ce procédé peut consister en n'importe quel traitement. De préférence, il s'agit de l'application topique d'une molécule, par enduction ou bien par injection, de préférence sous-cutanée. La molécule appliquée peut être une entité chimique simple ou la combinaison ou l'association de différentes entités, il peut s'agir de principes actifs, de molécules naturelles, de protéines, d'ARN, d'ADN, d'huile essentielle, d'entité chimique issue d'extraits naturels, d'une formulation complexe, d'une molécule d'ADN, d'un microARN, d'un siRNA, d'un inhibiteur de miRNA, notamment d'un antagomir, etc...

Des molécules particulièrement préférées dans le cadre de traitement à tester selon l'invention, sont notamment les ARN, tout particulièrement des microARNs, ou des molécules modifiant l'expression de microARNs naturels, tels que des inhibiteurs de miRNA et notamment les antagomirs.

Il peut s'agir également de l'application d'un rayonnement électromagnétique, quelque soit la longueur d'onde, par exemple de longueur d'ondes visibles, UV, IR ou X. Le traitement au sens de la présente invention englobe également l'application de différents effets mécaniques.

Le procédé de l'invention est considéré comme efficace s'il engendre une modification du niveau d'expression d'au moins un des microARNs de l'invention au sein de l'épithélium sous étude. De préférence, ledit traitement est considéré comme efficace s'il engendre la modification du niveau d'expression d'au moins deux, voire d'au moins cinq desdits microARNs au sein de l'épithélium. Ledit traitement peut engendrer une modification du niveau d'expression d'au moins 10, 15 ou même des 19 microARNs de l'invention. Les modifications engendrées sur le niveau d'expression des microARNs de l'invention ne sont pas nécessairement d'amplitude identique pour tous les microARNs.

De préférence, au sens de la présente invention, un traitement testé sera considéré comme efficace s'il engendre une modification du niveau d'expression d'au moins un microARN choisi parmi hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b concomitamment avec une modification du niveau d'expression dans le sens inverse à la précédente d'au moins un microARN choisi parmi hsa-let-7i, hsa-miR-22, hsa-miR-221, hsa-miR-222, hsa-miR-29a, hsa-miR-29b, hsa-miR-663, hsa-miR-30a et hsa-miR-30c. Ainsi un traitement efficace au sens de l'invention engendrera par exemple une augmentation du niveau d'expression d'au moins un microARN choisi parmi hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, et, simultanément, également la diminution du niveau d'expression d'au moins un microARN choisi parmi hsa-let-7i, hsa-miR-22, hsa-miR-221, hsa-miR-222, hsa-miR-29a, hsa-miR-29b, hsa-miR-663, hsa-miR-30a et hsa-miR-30c.

Il est également possible, par les méthodes de détermination de l'efficacité d'un traitement de la présente demande, d'objectiver l'action bénéfique d'un agent, notamment d'un produit cosmétique, par exemple d'une composition cosmétique, quant à son action sur l'état de différenciation épidermique de la peau. Les méthodes de détermination décrites plus haut in vitro peuvent en effet être utilisées dans un protocole de test permettant de déterminer les produits susceptibles d'être qualifiés d'agent ayant un effet sur l'état prolifératif ou différencié des kératinocytes de la peau.

Par ailleurs, au moyen de ce test, il est possible de promouvoir le produit auprès de consommateurs, en mettant en avant les résultats obtenus avec ce produit dans les méthodes de détermination décrites dans la présente invention. L'évaluation de l'effet sur l'état de différenciation épidermique sera basée sur l'étude de l'expression des microARNs de l'invention. La présente description divulgue donc également une méthode permettant de recommander un produit en signalant son effet dans un protocole de test constitué par une méthode de détermination telle que décrite précédemment. La description divulgue donc également une méthode pour la promotion d'un produit cosmétique consistant à faire état d'une efficacité, action ou propriété dudit produit démontrée par au moins un test opéré tel que décrit précédemment.

Une telle promotion du produit pourra se faire par n'importe quel canal de communication. Elle pourra être faite notamment par la vendeuse, directement sur le point de vente, par la radio et la télévision, notamment dans le cadre de spots publicitaires. Elle pourra être faite également par le canal de la presse écrite, ou par le biais de tout autre document, en particulier à des fins publicitaires (prospectus). Elle pourra se faire également par Internet, ou par tout autre réseau informatique adéquat. Elle pourra être faite également directement sur le produit, notamment sur son packaging ou sur toute notice explicative qui peut lui être associée.

La présente invention, selon un **troisième** aspect, concerne également divers procédés de criblage de molécules pour leur action sur le processus de différenciation épidermique. Notamment, l'invention concerne un procédé de criblage de modulateurs du processus de différenciation épidermique au sein d'un épithélium, comprenant l'évaluation de l'état de différenciation épidermique de kératinocytes au sein de cet épithélium, avant et après application du modulateur à cribler.

De tels procédés de criblage sont mis en oeuvre ex-vivo, ou in vitro, par exemple sur des épithéliums de culture ou des échantillons d'épithélium.

L'évaluation de l'état de différenciation épidermique de kératinocytes au sein de cet épithélium est réalisée par une des méthodes selon le premier aspect de l'invention.

Comme précisé dans ce qui précède, l'épithélium auquel il est fait référence peut être un épithélium de culture, par exemple un épithélium reconstitué, type « peau reconstruite » ou bien un échantillon obtenu d'un être humain, par exemple lors d'une biopsie.

Alternativement, la description divulgue également un procédé de criblage de modulateurs du processus de différenciation épidermique au sein d'un épithélium, comprenant l'évaluation du différentiel de différenciation épidermique de cet épithélium, avant et après application du modulateur à cribler.

L'évaluation du différentiel de différenciation épidermique de kératinocytes au sein de cet épithélium est réalisée par une des méthodes selon le premier aspect de l'invention.

Le modulateur dont il est question peut être toute entité chimique, par exemple issue d'extraits naturels ou bien de synthèse, il peut s'agir d'une formulation complexe, d'une molécule d'ADN, d'un microARN, d'un siRNA ou d'un inhibiteur de miRNA tel qu'un antagomir par exemple. Cette liste n'est bien entendu pas exhaustive.

Alternativement, le modulateur peut être un système instrumental utilisant un rayonnement électromagnétique, de préférence un rayonnement d'ondes visibles, UV, IR ou X, ou bien un système utilisant des effets mécaniques.

Selon un **quatrième** aspect, la présente invention concerne également un procédé de diagnostic de l'état d'un épiderme, grâce à l'utilisation de la signature microARN mise en évidence par les inventeurs. Un tel procédé de diagnostic comprend la détermination du différentiel de différenciation épidermique existant au sein de cet épiderme entre la couche basale et la couche cornée, et la comparaison au différentiel de différenciation épidermique moyen existant au sein d'un épiderme normal de même type, entre la couche basale et la couche cornée.

La détermination du différentiel de différenciation épidermique est effectuée à l'aide des différentes méthodes selon le premier aspect de l'invention. Elle est effectuée sur un échantillon d'épiderme de la peau à diagnostiquer.

Le différentiel de différenciation épidermique moyen existant au sein d'un épiderme normal de même type, entre la couche basale et la couche cornée, correspond à une valeur moyenne de plusieurs mesures effectuée sur plusieurs épidermes provenant de sujets dont la peau est saine et de même type que celle de l'épiderme à diagnostiquer. Par même type, on entend âge comparable, ethnie et couleurs identiques. Il s'agit d'une valeur moyenne qui correspond à un état « normal » ou « sain », exempt de toute pathologie ou lésion de l'épiderme.

Par ce procédé de diagnostic, il est ainsi établi le profil microARNs de la peau à diagnostiquer et par comparaison, il peut être diagnostiqué éventuellement un vieillissement prématuré, un photo-vieillissement ou des dommages causés par le rayonnement solaire, un vieillissement ou des dommages causés par le stress, l'acné, ou tout autre trouble physiologique ou pathologique.

Selon encore un **autre** aspect, la présente demande a aussi pour objet l'utilisation du dosage de l'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c, dans des kératinocytes au sein d'un échantillon d'épithélium humain pour la détermination de l'état de différenciation épidermique desdits kératinocytes.

En effet, les présents inventeurs ont montré que ce dosage donne accès à la signature microARN de la différenciation épidermique.

Selon encore un **autre** aspect, la description divulgue diverses utilisations, cosmétiques et en thérapie, des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c, ou de modulateurs de l'expression desdits microARNs, et tout particulièrement des inhibiteurs de miRNA, tels que les antagomirs .

En effet, du fait de l'importance de ces microARNs dans le processus de différenciation épidermique de la peau, la modulation de leur niveau au sein de kératinocytes est susceptible d'influencer la différenciation épidermique, par exemple de la ralentir ou au contraire de l'accélérer afin de ralentir ou d'accélérer le processus de renouvellement de la couche cornée. La modulation du niveau des microARNs de l'invention au sein de kératinocytes est de manière toute préférentielle effectuée en introduisant des microARNs de l'invention, afin d'en augmenter leur niveau au sein des kératinocytes, notamment, l'utilisation de microARNs pour corriger des dysrégulations endogènes des microARNs. La modulation peut également être réalisée par l'introduction de modulateurs desdits microARNs, de préférence il s'agit de l'introduction d'inhibiteurs de miRNA, par exemple d'antagomirs, ciblant des microARNs de l'invention afin d'en diminuer leur niveau au sein des kératinocytes. D'autres inhibiteurs de miRNA tout particulièrement envisagés dans le cadre de cette invention sont notamment les miRCURY LNA™ microRNA Knockdown Probes (Exiqon) et les miScript miRNA inhibitors de Qiagen.

La description divulgue un microARN choisi parmi les 19 microARNs de l'invention, ou bien un modulateur de ce microARN, notamment un inhibiteur de miRNA, tel qu'un antagomir, ciblant ce microARN, pour une utilisation thérapeutique pour traiter des pathologies cutanées associées au programme de différenciation épidermique. Les pathologies concernées sont de préférence des pathologies générant des dysfonctionnements de la différenciation épidermique.

Des pathologies tout particulièrement considérées dans le cadre de cette divulgation sont notamment le psoriasis, la dermatite atopique, l'acné, les ichthyoses, le syndrome de Netherton, le pityriasis rubra pilaire, la kératose pilaire, la maladie de Darier, les kératodermies palmoplantaires et les porokératoses.

La description divulgue des compositions comprenant au moins deux, voire trois, de préférence au moins 5, 10, 15 ou bien les 19 microARNs de l'invention, pour les applications thérapeutiques visées plus haut, ou bien des inhibiteurs de miRNA, notamment des antagomirs d'au moins plusieurs de ces microARNs.

Concernant les applications thérapeutiques, si l'effet thérapeutique souhaité consiste en l'augmentation de l'état de différenciation épidermique de la peau à traiter, la description divulgue des compositions comprenant les microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, pour une utilisation en thérapie, de préférence pour traiter des pathologies cutanées associées au programme de différenciation épidermique, notamment pour traiter le psoriasis, la dermatite atopique, l'acné, les ichthyoses , le syndrome de Netherton, le pityriasis rubra pilaire, la kératose pilaire, la maladie de Darier, les kératodermies palmoplantaires et les porokératoses. Les compositions comprennent en sus des microARNs cités, des inhibiteurs de miRNA, tels que des antagomirs, ciblant les microARNs hsa-let-7i, hsa-miR-22, hsa-miR-221, hsa-miR-222, hsa-miR-29a, hsa-miR-29b, hsa-miR-663, hsa-miR-30a et hsa-miR-30c, pour une utilisation dans le traitement desdites pathologies. D'autres inhibiteurs de miRNA tout particulièrement envisagés dans le cadre de cette invention sont notamment les miRCURY LNA™ microRNA Knockdown Probes (Exiqon) et les miScript miRNA inhibitors de Qiagen.

Pour les situations nécessitant une stimulation de la différenciation terminale, une composition ou produit pour l'application thérapeutique mentionnée, comprend donc :
- l'ensemble des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, ou bien des modulateurs de ces microARNs entraînant une augmentation de leur expression ; et
- des inhibiteurs de miRNA, tels que des antagomirs, ciblant les microARNs hsa-let-7i, hsa-miR-22, hsa-miR-221, hsa-miR-222, hsa-miR-29a, hsa-miR-29b, hsa-miR-663, hsa-miR-30a et hsa-miR-30c, ou bien des modulateurs de ces microARNs entraînant une répression de leur expression.

Des inhibiteurs de miRNA tout particulièrement envisagés dans le cadre de cette invention sont notamment les antagomirs, les miRCURY LNA™ microRNA Knockdown Probes (Exiqon) et les miScript miRNA inhibitors de Qiagen.

A l'inverse, si l'effet thérapeutique souhaité est par exemple de ralentir la différenciation épidermique, ou bien de favoriser le potentiel prolifératif de kératinocytes de la peau traitée, la description divulgue des compositions comprenant les microARNs hsa-let-7i, hsa-miR-22, hsa-miR-221, hsa-miR-222, hsa-miR-29a, hsa-miR-29b, hsa-miR-663, hsa-miR-30a et hsa-miR-30c, pour une utilisation en thérapie, de préférence pour traiter des pathologies cutanées associées au programme de différenciation épidermique, notamment pour traiter le psoriasis, la dermatite atopique, l'acné, les ichthyoses, le syndrome de Netherton, le pityriasis rubra pilaire, la kératose pilaire, la maladie de Darier, les kératodermies palmoplantaires et les porokératoses. Les compositions comprennent en sus des microARNs cités, des inhibiteurs de miRNA, par exemple des antagomirs, ciblant les microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, pour une utilisation dans le traitement desdites pathologies.

Pour les situations nécessitant une réduction de la différenciation terminale, une composition ou produit pour l'application thérapeutique mentionnée comprend donc :
- l'ensemble des microARNs hsa-let-7i, hsa-miR-22, hsa-miR-221, hsa-miR-222, hsa-miR-29a, hsa-miR-29b, hsa-miR-663, hsa-miR-30a et hsa-miR-30c, ou bien des modulateurs de ces microARNs entraînant une augmentation de leur expression ; et
- des inhibiteurs de miRNA, par exemple des antagomirs, ciblant les microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, ou bien des modulateurs de ces microARNs entraînant une répression de leur expression.

Des inhibiteurs de miRNA tout particulièrement envisagés dans le cadre de cette invention sont notamment les antagomirs, les miRCURY LNA™ microRNA Knockdown Probes (Exiqon) et les miScript miRNA inhibitors de Qiagen.

De préférence, les compositions pour une utilisation en thérapie sont formulées avec des excipients pharmaceutiquement acceptables, pour une application topique ou par ingestion.

La présente description divulgue également des compositions cosmétiques comprenant les microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c, ou bien un modulateur desdits microARNs, de préférence un inhibiteur de miRNA, tel qu'un antagomir, ciblant l'un desdits microARNs, notamment pour des applications cosmétiques sur une peau humaine non pathologique.

Les différentes utilisations mentionnées en thérapie ou en cosmétique, impliquent de préférence l'application topique, locale de microARNs ou de composition comprenant lesdits microARNs, ou des inhibiteurs desdits miRNAs, par exemple des antagomirs. Du fait de leur petite taille, les microARNs et leurs inhibiteurs sont en effet susceptibles de traverser la membrane des kératinocytes et de modifier le niveau desdits microARNs au sein des kératinocytes traités. Par ailleurs, du fait de leur nature physico-chimique, il est peu probable qu'ils atteignent les couches profondes de la peau, limitant donc leur action à l'épiderme.

La stabilité des microARNs et de leurs inhibiteurs, notamment antagomirs, laisse penser que leur action est transitoire, et la présente description divulgue donc des utilisations en thérapie ou en cosmétique impliquant des applications renouvelées sur la peau à traiter.

De ce fait, la description divulgue notamment une méthode de traitement de troubles liés à la différenciation épidermique dans des situations cosmétiques physiologiques, non pathologiques, comprenant l'application topique sur la peau d'un individu d'un microARN choisi parmi hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c et / ou d'un modulateur de l'expression d'un de ces microARNs, de préférence d'un inhibiteur de miRNA, par exemple d'un antagomir ciblant l'un de ces microARNs.

Les troubles de la différenciation épidermique dans des situations cosmétiques sont liés notamment à l'âge, à la dermatohéliose, à la sécheresse cutanée ou xérose, ou à des agressions chimiques, notamment par des agents exfoliants, desquamants, délipidants, ou des agressions mécaniques. Ces troubles ne sont pas pathologiques et leur traitement n'est pas à considérer comme un traitement thérapeutique, mais uniquement un traitement à visée cosmétique, le traitement de ces troubles ayant pour but essentiel d'améliorer l'apparence de la peau et le confort de la personne.

Plus spécifiquement, les troubles liés à l'âge consistent en des modifications de la différenciation épidermique avec le vieillissement chronologique. En effet, lors du processus de vieillissement, l'épiderme subit de nombreuses transformations ayant des répercutions sur le processus de différenciation épidermique et la formation de couches cornées fonctionnelles. On notera en particulier un amincissement de l'épiderme, avec un nombre plus restreint de couches suprabasales et une diminution du compartiment prolifératif avec une diminution du nombre de cellules clonogéniques, et donc également du turnover épidermique. Les marqueurs de différenciation classiques, tels que le profil d'expression des kératines, est modifié avec une expression des kératines 16 et 17 non trouvée dans un épiderme jeune et sain. Les taux des kératines K1 et K15 sont diminués, ainsi que celui de la filaggrine, principale composante des couches granuleuses. Le taux de transglutaminase1 a aussi été trouvé diminué avec l'âge. La fonction barrière est altérée avec une perméabilité accrue.

Les troubles liés à la dermatohéliose, ou 'photo-aging', s'entendent de modifications de la différenciation épidermique avec l'exposition chronique à la lumière solaire. La peau dite « photo-âgée » est caractérisée notamment par l'aspect de cuir tanné, et la présence de rides plus prononcées, cette peau est également modifiée au niveau épidermique, avec une surexpression des kératines K6, K16, K17 et une diminution de la filaggrine et de la transglutaminase. Ces altérations ont été associées à la formation des rides. Les protéines SPRR impliquées dans la formation des couches cornées sont aussi altérées par les UV. Il est aussi connu que l'exposition aiguë au soleil entraîne des modifications épidermiques affectant la fonction barrière et l'homéostasie épidermique.

Les troubles liés à la sécheresse ou xérose sont principalement dus au fait qu'une peau sèche se caractérise essentiellement par une diminution du contenu lipidique de l'épiderme et une altération du stratum corneum. Les kératines 1 et 10 sont diminuées et les kératines K5 et K14 augmentées. Le niveau d'expression de la filaggrine est perturbé, de même que celui de l'involucrine.

Les agressions chimiques, notamment par des agents exfoliants, desquamants, délipidants, et les agressions mécaniques sont responsables de modifications de l'homéostasie épidermique et induisent une altération de la fonction barrière assurée par le stratum corneum. Le processus de différenciation épidermique normal permet de rétablir une homéostasie épidermique et une fonction barrière normale.

Par l'application, de préférence topique, des microARNs de l'invention, ou d'inhibiteurs spécifiques de ces miRNAs, il est possible de traiter les différents troubles non pathologiques mentionnés ci-dessus, dans des applications cométiques.

La présente description divulgue également des compositions ou produits cosmétiques, comprenant au moins l'un des microARNs de l'invention ou bien comprenant au moins un modulateur de l'expression d'un des microARNs de l'invention, pour corriger ou améliorer l'aspect de la peau, son grain, son éclat, ses imperfections ou son microrelief. Un modulateur de l'expression d'un des microARNs de l'invention est un actif entraînant l'augmentation ou la répression de l'expression d'un desdits microARNs. Un tel actif modulateur de l'expression d'un des microARNs est par exemple identifié par les procédés de criblage décrits précédemment, ou bien il s'agit d'un antagomir d'un microARN de l'invention.

Le produit ou composition cosmétique dont il est question peut être sous toute forme galénique, par exemple formulé sous forme de lait, de crème, de gel, de spray ou de savon, pour être administré par voie topique, ou bien sous forme ingérable pour la voie orale.

Il peut notamment s'agir d'une combinaison de différents actifs, par exemple une combinaison d'au moins deux microARNs de l'invention, ou d'un microARN de l'invention et d'un modulateur de microARN de l'invention, ou bien de deux modulateurs des microARNs de l'invention. Il peut également s'agir de la combinaison d'un actif et d'un instrument, notamment d'un instrument de type traitement mécanique, lumineux, électrique ou électromagnétique.

Dans le cadre de la présente description, il est notamment divulgué des produits cosmétiques dits « anti-âge » pour réduire les imperfections de surface liées aux désordres de la différenciation et l'aspect parcheminé de la peau âgée. Il est également envisagé des produits cosmétiques spécifiques pour les peaux âgées exposées au soleil, en restaurant une meilleure qualité, et ce en modifiant la balance entre prolifération et différenciation épidermique, grâce aux microARNs de l'invention ou à des modulateurs desdits microARNs.

La description divulgue également des produits cosmétiques après-soleil, pour contrebalancer les modifications de la différenciation induites par l'exposition aiguë au soleil. Sont également divulgués des produits permettant de réguler les désordres pigmentaires en stimulant la différenciation épidermique et la desquamation kératinocytaire afin d'éliminer plus rapidement la surcharge de mélanine responsable du désordre pigmentaire.

D'autres produits cosmétiques sont également des produits spécifiquement élaborés pour les peaux sèches, qui aient un effet hydratant, également en modulant les étapes terminales de la différenciation épidermique. Des produits réparateurs pour améliorer le processus de réparation cutanée lors de la cicatrisation sont également envisagés dans le cadre de cette invention. Il est également possible de formuler des produits destinés à des peaux agressées, soit dans le cadre de procédures spécifiques (produits post-peelings, post-lasers,...), soit dans des situations quotidiennes, telles que par la pollution, les produits ménagers, etc...

Pour les situations nécessitant une stimulation de la différenciation terminale, afin d'augmenter la fonction barrière et la couche cornée, une composition ou produit cosmétique au sens de la présente description comprend :
- au moins un, et de préférence, au moins 2, 3, 5 ou l'ensemble des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, ou bien des modulateurs de ces microARNs entraînant une augmentation de leur expression ; et/ou éventuellement
- des inhibiteurs des microARNs hsa-let-7i, hsa-miR-22, hsa-miR-221, hsa-miR-222, hsa-miR-29a, hsa-miR-29b, hsa-miR-663, hsa-miR-30a et hsa-miR-30c, ou bien des modulateurs de ces microARNs entraînant une répression de leur expression. Des inhibiteurs de miRNA tout particulièrement envisagés dans le cadre de cette invention sont notamment les antagomirs, les miRCURY LNATM microRNA Knockdown Probes (Exiqon) et les miScript miRNA inhibitors de Qiagen.

Pour les situations nécessitant une réduction de la différenciation terminale et une diminution de la formation de couches cornées, une composition ou produit cosmétique au sens de la présente description comprend :
- au moins un, et de préférence, au moins 2, 3, 5 ou l'ensemble des microARNs hsa-let-7i, hsa-miR-22, hsa-miR-221, hsa-miR-222, hsa-miR-29a, hsa-miR-29b, hsa-miR-663, hsa-miR-30a et hsa-miR-30c, ou bien des modulateurs de ces microARNs entraînant une augmentation de leur expression ; et/ ou éventuellement
- des inhibiteurs des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, ou bien des modulateurs de ces microARNs entraînant une répression de leur expression. Des inhibiteurs de miRNA tout particulièrement envisagés dans le cadre de cette invention sont notamment les antagomirs, les miRCURY LNATM microRNA Knockdown Probes (Exiqon) et les miScript miRNA inhibitors de Qiagen.

La présente invention a aussi pour objet un kit pour la détermination de l'état de différenciation d'un kératinocyte humain, comprenant des moyens pour le dosage de l'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c et la mention d'un niveau de référence pour l'expression desdits microARNs.

Un kit particulièrement préféré dans le cadre de la présente invention est un kit tel que le niveau de référence est le niveau d'expression moyen desdites microARN dans des kératinocytes en culture non différenciés.

Alternativement, il peut être fait usage dans un kit selon l'invention d'un niveau de référence qui corresponde au niveau d'expression moyen desdites microARN dans des kératinocytes épidermiques différenciés.

Les différentes mises en oeuvre préférées décrites plus spécifiquement pour l'un ou l'autre des aspects de l'invention, sont également applicables aux autres aspects de l'invention.

### Partie Expérimentale :

### MATERIEL ET METHODES

### Culture de kératinocytes humains en monocouche

Les kératinocytes épidermiques humains normaux sont issus de plastie mammaire et sont cultivés classiquement selon la méthode de Rheinwald et Green, sur une couche nourricière de fibroblastes Swiss 3T3 préalablement mitomycinés.

### Peaux reconstruites

Des peaux reconstruites *in vitro* comprenant un épiderme stratifié et différencié avec des couches cornées et un derme équivalent vivant, sont réalisées avec des kératinocytes humains normaux et des fibroblastes humains normaux de derme.

Brièvement, des dermes équivalents sont réalisés avec du collagène I bovin et un million de fibroblastes humains de derme. Les kératinocytes normaux sont ensemencés après contraction des lattices à raison de 500000 kératinocytes et cultivés dans le milieu classique pendant 7 jours. La culture est ensuite montée sur grille pour la phase d'émersion (interface air-liquide, 7 jours).

A la fin de cette période, les peaux présentent une morphologie très proche de la peau humaine normale. Elles peuvent être alors utilisées pour différentes expériences en maintenant le milieu de culture sous la peau qui est nourrie par capillarité.

### Extraction des ARN totaux des kératinocytes

### Lyse cellulaire:

Les kératinocytes en culture monocouche sont lysés sur boite de culture dans le tampon Tri Reagent.

Les épidermes reconstruits des peaux reconstruites sont séparés du derme équivalent à l'aide de pinces. Ces épidermes sont broyés manuellement dans des potters en verre, en tampon de lyse Tri Reagent.

### Extraction des ARN totaux

Les ARN totaux sont extraits par la technique classique de lyse en solution de guanidium isothiocyanate puis extraction grâce à un mélange phénol/chloroforme.

### Hybridation des microARN sur les puces µParaflo^{™} « MiRHuman 10.0 ».

Les petits ARN sont extraits des ARN totaux (filtre Microcon, Millipore) et sont étendus en 3' avec une queue polyA. Un oligonucléotide marqué en fluorescence est ligué à la queue polyA. Les petits ARN marqués sont ensuite hybridés sur les puces µParaflo^{™} « MiRHuman 10.0 » (Atactic Technologies, LC Sciences). Elles contiennent les séquences complémentaires de tous les microARN humains répertoriés dans miRBase 10.0 (711 microARN matures). Les séquences ont été déposées 5 fois sur la puce. La fluorescence est quantifiée et les signaux sont normalisés. Le taux de fluorescence reflète le taux d'expression de chaque microARN dans l'échantillon.

### Analyse statistique

L'expression de chaque microARN est comparée statistiquement entre l'échantillon « kératinocytes en culture » et l'échantillon « kératinocyte en épiderme reconstruit » en utilisant le test t de student (p<0.05).

### EXEMPLE 1 : Liste des 10 microARN sur-exprimés dans les kératinocytes d'épiderme reconstruit différencié par rapport aux kératinocytes en culture monocouche undifférenciés.

Trois souches de kératinocytes humains normaux primaires issus de plasties mammaires ont été utilisées dans l'étude (PH202, PH64 et PH63). L'expression de tous les microARN humains connus a été quantifiée, dans les kératinocytes cultivés en monocouche, c'est-à-dire des kératinocytes non différenciés (2D) et en parallèle dans des épidermes reconstruits avec ces mêmes kératinocytes (3D).

L'expression de chaque microARN est comparée statistiquement entre l'échantillon « kératinocytes en culture » et l'échantillon « kératinocyte en épiderme reconstruit » en utilisant le test t de student qui renvoie la probabilité p. Chaque microARN cité dans le tableau 1 a un niveau statistiquement plus élevé dans l'épiderme par rapport aux kératinocytes non différenciés (p<0 ,05), et ce pour chaque souche cellulaire étudiée. Le ratio d'expression 3D/2D est donné pour chaque souche. Par exemple miR-141 est en moyenne 2,3 fois plus abondant dans les kératinocytes issus d'épiderme reconstruit que dans les kératinocytes cultivés en monocouche.

**Tableau 1 : microARN sur-exprimés dans les kératinocytes d'épiderme reconstruit (3D) c'est-à-dire différenciés, par rapport aux kératinocytes en culture monocouche (2D), c'est-à-dire non différenciés.**

| Kératinocytes | | 2D | | 3D | | | | |
|---|---|---|---|---|---|---|---|---|
| Nom du microARN | | Signal moyen | Déviation standard | Signal moyen | Déviation standard | p_value | Ratio 3D/2D | Ratio moyen 3D/2D |
| **hsa-miR-141** | PH202 | **1047** | 127 | **1930** | 491 | 0,004 | 1,8 | |
| | PH64 | **526** | 68 | **1405** | 86 | 0,00002 | 2,7 | **2,3** |
| | PH63 | **916** | 111 | **2079** | 387 | 0,0002 | 2,3 | |
| **hsa-miR-148a** | PH202 | **214** | 39 | **619** | 50 | 0,00008 | 2,9 | |
| | PH64 | **127** | 37 | **703** | 53 | 0,0002 | 5,5 | **4,1** |
| | PH63 | **117** | 21 | **466** | 49 | 0,000004 | 4,0 | |
| **hsa-miR-182** | PH202 | **538** | 80 | **922** | 94 | 0,0002 | 1,7 | |
| | PH64 | **519** | 63 | **873** | 116 | 0,0003 | 1,7 | **1,6** |
| | PH63 | **530** | 38 | **724** | 58 | 0,0003 | 1,4 | |
| **hsa-miR-224** | PH202 | **513** | 108 | **805** | 245 | 0,02 | 1,6 | |
| | PH64 | **574** | 164 | **1085** | 278 | 0,005 | 1,9 | **1,8** |
| | PH63 | **393** | 90 | **723** | 138 | 0,002 | 1,8 | |
| **hsa-miR-26a** | PH202 | **4673** | 495 | **7757** | 610 | 0,00007 | 1,7 | |
| | PH64 | **4370** | 704 | **7025** | 681 | 0,001 | 1,6 | **1,6** |
| | PH63 | **4616** | 589 | **6707** | 346 | 0,002 | 1,5 | |
| **hsa-miR-26b** | PH202 | **820** | 106 | **1110** | 82 | 0,004 | 1,4 | |
| | PH64 | **654** | 175 | **1390** | 154 | 0,002 | 2,1 | **2,0** |
| | PH63 | **297** | 67 | **750** | 80 | 0,0005 | 2,5 | |
| **hsa-miR-361-5p** | PH202 | **337** | 46 | **723** | 37 | 0,0001 | 2,1 | |
| | PH64 | **505** | 88 | **1269** | 99 | 0,0001 | 2,5 | **2,3** |
| | PH63 | **369** | 58 | **853** | 36 | 0,0003 | 2,3 | |
| **hsa-miR-425** | PH202 | **339** | 16 | **559** | 41 | 0,00002 | 1,6 | |
| | PH64 | **416** | 71 | **551** | 67 | 0,02 | 1,3 | **1,4** |
| | PH63 | **502** | 44 | **581** | 22 | 0,02 | 1,2 | |
| **hsa-miR-455-3p** | PH202 | **299** | 38 | **1053** | 96 | 0,0000005 | 3,5 | |
| | PH64 | **284** | 64 | **1265** | 187 | 0,00003 | 4,5 | **3,7** |
| | PH63 | **325** | 50 | **1012** | 83 | 0,00001 | 3,1 | |
| **hsa-miR-92b** | PH202 | **1104** | 107 | **1632** | 112 | 0,0002 | 1,5 | |
| | PH64 | **1487** | 252 | **2400** | 150 | 0,001 | 1,6 | **1,5** |
| | PH63 | **1482** | 129 | **1906** | 135 | 0,002 | 1,3 | |

### EXEMPLE 2 : Liste des 9 microARN sur exprimés dans les kératinocytes en culture monocouche undifférenciés par rapport aux kératinocytes d'épiderme reconstruit différenciés.

3 souches de kératinocytes humains normaux primaires issus de plasties mammaires ont été utilisées dans l'étude (PH202, PH64 et PH63). L'expression de tous les microARN humains connus a été quantifiée, dans les kératinocytes cultivés en monocouche, c'est-à-dire des kératinocytes non différenciés et en parallèle dans des épidermes reconstruits avec ces mêmes kératinocytes. L'expression de chaque microARN est comparée statistiquement entre l'échantillon « kératinocytes en culture » et l'échantillon « kératinocyte en épiderme reconstruit » en utilisant le test t de student qui renvoie la probabilité p. Chaque microARN cité dans ce tableau a un niveau statistiquement plus élevé dans les kératinocytes non différenciés par rapport aux kératinocytes d'épiderme reconstruit (p<0 ,05), et ce pour chaque souche cellulaire étudiée.

Le ratio d'expression 2D/3D est donné pour chaque souche. Par exemple miR-29b est en moyenne 6,7 fois plus abondant dans les kératinocytes cultivés en monocouche que dans les kératinocytes d'épiderme reconstruit.

**Tableau 2 : microARN sur exprimés dans les kératinocytes en culture monocouche (2D), c'est-à-dire non différenciés, par rapport aux kératinocytes d'épiderme reconstruit (3D), c'est-à-dire différenciés.**

| | **Kératinocytes** | **2D** | | **3D** | | | | |
|---|---|---|---|---|---|---|---|---|
| Nom du microARN | | Signal moyen | Déviation standard | Signal moyen | Déviation standard | p_value | Ratio 2D/3D | Ratio moyen 2D/3D |
| **hsa-let-7i** | PH202 | **2947** | 77 | **1211** | 111 | 0,00003 | 2,4 | |
| | PH64 | **2573** | 297 | **1208** | 157 | 0,00002 | 2,1 | **2,4** |
| | PH63 | **3125** | 179 | **1178** | 90 | 0,0000001 | 2,7 | |
| **hsa-miR-22** | PH202 | **2668** | 241 | **2316** | 96 | 0,03 | 1,2 | |
| | PH64 | **2497** | 436 | **1645** | 155 | 0,004 | 1,5 | **1,4** |
| | PH63 | **3661** | 361 | **2386** | 155 | 0,0002 | 1,5 | |
| **hsa-m i R-221** | PH202 | **5556** | 792 | **3184** | 175 | 0,0005 | 1,7 | |
| | PH64 | **3213** | 774 | **1844** | 117 | 0,01 | 1,7 | **2,0** |
| | PH63 | **5558** | 360 | **2321** | 75 | 0,000001 | 2,4 | |
| **hsa-miR-222** | PH202 | **6069** | 637 | **3022** | 195 | 0,00002 | 2,0 | |
| | PH64 | **3901** | 303 | **1867** | 106 | 0,000001 | 2,1 | **2,4** |
| | PH63 | **7763** | 505 | **2580** | 170 | 0,00000003 | 3,0 | |
| **hsa-miR-29a** | PH202 | **7593** | 1191 | **3826** | 357 | 0,00023 | 2,0 | |
| | PH64 | **9569** | 1407 | **2624** | 550 | 0,00001 | 3,6 | **3,0** |
| | PH63 | **11055** | 291 | **3258** | 272 | 0,00001 | 3,4 | |
| **hsa-miR-29b** | PH202 | **1070** | 116 | **192** | 13 | 0,0000001 | 5,6 | |
| | PH64 | **734** | 136 | **96** | 40 | 0,0002 | 7,7 | **6,7** |
| | PH63 | **947** | 60 | **135** | 18 | 0,0000007 | 7,0 | |
| **hsa-m**i**R-663** | PH202 | **1537** | 157 | **564** | 31 | 0,000002 | 2,7 | |
| | PH64 | **1090** | 199 | **691** | 129 | 0,008 | 1,6 | **2,0** |
| | PH63 | **2053** | 241 | **1233** | 110 | 0,0001 | 1,7 | |
| **hsa-m**i**R-30a** | PH202 | **707** | 46 | **404** | 28 | 0,000004 | 1,7 | |
| | PH64 | **787** | 104 | **504** | 147 | 0,02 | 1,6 | **1,9** |
| | PH63 | **901** | 89 | **374** | 38 | 0,000003 | 2,4 | |
| **hsa-m**i**R-30c** | PH202 | **1667** | 111 | **990** | 77 | 0,00001 | 1,7 | |
| | PH64 | **2260** | 325 | **1315** | 115 | 0,0004 | 1,7 | **1,6** |
| | PH63 | **1619** | 216 | **1215** | 109 | 0,009 | 1,3 | |

### EXEMPLE 3 : Méthodes de détection des miRNAs.

### Northern Blot :

Cette technique classique est qualitative et quantitative. Elle permet l'analyse de quelques miRNAs lors d'une expérimentation. Cette méthode requiert l'utilisation de 5 à 50 µg d'ARN total.

### RT Q-PCR

Cette méthode permet l'étude des précurseurs de miRNAs et a été adaptée pour l'étude des miRNAs matures. Celle-ci est commercialisée (Applied Biosystems TaqMan MicroRNA Assays) et est décrite dans plusieurs articles [23].

### miRNA Microarray

Cette méthode, comme celle des microarrays pour ARNm classiques, est basée sur le marquage fluorescent des ADNc complémentaires des miRNAs d'un échantillon, et l'hybridation des ces ANDc marqués sur une lame sur laquelle sont déposées au préalable des séquences de miRNAs connus [25].

D'après certains auteurs, cette technologie miRNA microarray, si elle intègre régulièrement les nouvelles séquences de miRNAs issues de miRBase, serait la méthode de choix pour l'analyse de profil global d'expression de miRNAs.

De plus, dans certaines situations comme la présente invention, le profil d'expression des miRNAs est plus informatif que le profil d'expression des ARN messagers.

### Hybridation in situ des microARNs

Cette technique est plus délicate que l'hybridation in situ classique des ARNm, car par la méthode de fixation classique, les petits ARNs diffusent plus que les longs ARNs et sont perdus lors de étapes d'hybridation et de lavage. Des techniques ont été mises au point pour pallier à cet inconvénient ; de plus, des sondes miRNAs sont disponibles dans le commerce (Exiqon, Denmark) [27].

### MiRNA transgène rapporteur

Dans cette technique, un gène reporteur GFP ou LacZ est placé sous le contrôle d'un promoteur incluant la partie 3'UTR complémentaire d'un miRNA.

### BIBLIOGRAPHIE

1. Bartel DP. MicroRNAs: genomics, biogenesis, mechanism, and function. Cell 2004; 116: 281-97.
3. Alvarez-Garcia I, Miska EA. MicroRNA functions in animal development and human disease. Development 2005; 132: 4653-62.
4. O'Donnell KA, Wentzel EA, Zeller KI et al. c-Myc-regulated microRNAs modulate E2F1 expression. Nature 2005; 435: 839-43.
5. Fazi F, Rosa A, Fatica A et al. A minicircuitry comprised of microRNA-223 and transcription factors NFI-A and C/EBPalpha regulates human granulopoiesis. Cell 2005; 123: 819-31.
8. Yi R, O'Carroll D, Pasolli HA et al. Morphogenesis in skin is governed by discrete sets of differentially expressed microRNAs. Nat Genet. 2006; 38: 356-62.
10. Wenguang Z, Jianghong W, Jinquan L et al. A subset of skin-expressed microRNAs with possible roles in goat and sheep hair growth based on expression profiling of mammalian microRNAs. OMICS. 2007; 11: 385-96.
11. Yi R, Poy MN, Stoffel M et al. A skin microRNA promotes differentiation by repressing 'stemness'. Nature 2008; 452: 225-9.
12. Sonkoly E, Wei T, Janson PC et al. MicroRNAs: Novel Regulators Involved in the Pathogenesis of Psoriasis? PLoS.ONE. 2007; 2: e610.
13. Felicetti F, Errico MC, Bottero L et al. The promyelocytic leukemia zinc finger-microRNA-221/-222 pathway controls melanoma progression through multiple oncogenic mechanisms. Cancer Res 2008; 68: 2745-54.
14. Ishida-Yamamoto A, Tanaka H, Nakane H et al. Inherited disorders of epidermal keratinization. J Dermatol Sci 1998; 18: 139-54.
15. Hoffjan S, Stemmler S. On the role of the epidermal differentiation complex in ichthyosis vulgaris, atopic dermatitis and psoriasis. Br J Dermatol 2007; 157: 441-9.
17. Ghadially R, Brown BE, Sequeira-Martin SM et al. The aged epidermal permeability barrier. Structural, functional, and lipid biochemical abnormalities in humans and a senescent murine model. J.Clin.Invest 1995; 95: 2281-90.
18. Harding CR. The stratum corneum: structure and function in health and disease. Dermatol Ther 2004; 17 Suppl 1: 6-15.
19. Marionnet C, Bernerd F, Dumas A et al. Modulation of gene expression induced in human epidermis by environmental stress in vivo. J Invest Dermatol 2003; 121: 1447-58.
23. Chen C, Ridzon DA et al. Real-time quantification of microRNAs by stem-loop RT-PCR. Nucleic Acids Res 2005; 33: e179.
25. Vagin VV et al. A distinct small RNA pathway silences selfish genetic elements in the germline. Science 2006; 313: 320-324.
27. Takada S et al. Mouse microRNA profiles determined with a new and sensitive cloning method. Nucleic Acids Res 2006; 34: e115.

## Revendications

1. Méthode de détermination de l'état de différenciation épidermique d'un kératinocyte humain au sein d'un échantillon d'épithélium humain isolé, comprenant
- la détermination du niveau d'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c au sein dudit kératinocyte et
- la comparaison du niveau d'expression des microARNs au niveau d'expression moyen de ces microARNs dans des kératinocytes en culture non différenciés, ou dans des kératinocytes épidermiques différenciés, de préférence provenant de la même souche ou de la même population.

2. Méthode selon la revendication 1, où ledit kératinocyte est considéré comme différencié si le niveau d'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b, est statistiquement supérieur au niveau d'expression moyen des microARNs correspondants dans des kératinocytes en culture non différenciés.

3. Méthode selon la revendication 1, où ledit kératinocyte est considéré comme non différencié si le niveau d'expression des microARNs hsa-let-7i, hsa-miR-22, hsa-miR-221, hsa-miR-222, hsa-miR-29a, hsa-miR-29b, hsa-miR-663, hsa-miR-30a et hsa-miR-30c, est statistiquement supérieur au niveau d'expression moyen des microARNs correspondants dans des kératinocytes épidermiques différenciés.

4. Méthode d'évaluation du différentiel de différenciation épidermique existant au sein d'un échantillon d'épithélium humain isolé, comprenant la comparaison, entre deux couches de kératinocytes dudit épithélium, du niveau d'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c.

5. Méthode selon la revendication 4, où le différentiel de différenciation épidermique entre les deux couches se **caractérise par** une expression plus importante des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 et hsa-miR-92b dans une couche par rapport à l'autre, d'un facteur supérieur à 1,3, de préférence supérieur à 1,5.

6. Méthode selon la revendication 4, où le différentiel de différenciation épidermique entre les deux couches se **caractérise par** une expression plus importante des microARNs hsa-let-7i, hsa-miR-22, hsa-miR-221, hsa-miR-222, hsa-miR-29a, hsa-miR-29b, hsa-miR-663, hsa-miR-30a et hsa-miR-30c dans une couche par rapport à l'autre, d'un facteur supérieur à 1,3, de préférence supérieur à 1,5.

7. Méthode selon l'une des revendications 1 à 6, où ledit échantillon provient d'une peau humaine ou d'un épithélium de culture type « peau reconstruite ».

8. Procédé de détermination de l'efficacité d'un traitement d'un épithélium, comprenant la comparaison, avant et après traitement, du niveau d'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c au sein de kératinocytes de cet épithélium, ledit procédé n'étant pas mis en oeuvre sur des êtres humains.

9. Procédé selon la revendication 8, où ledit traitement est considéré comme efficace s'il engendre une modification du niveau d'expression d'au moins un, de préférence d'au moins deux ou cinq desdits microARNs au sein de l'épithélium.

10. Procédé de criblage *in vitro* ou *ex vivo* de modulateurs du processus de différenciation épidermique au sein d'un épithélium, comprenant l'évaluation de l'état de différenciation épidermique de kératinocytes au sein de cet épithélium par une méthode selon l'une des revendications 1 à 3, avant et après application du modulateur à cribler.

11. Procédé de diagnostic de l'état d'un épiderme, comprenant :
- la détermination du différentiel de différenciation épidermique existant au sein de cet épiderme entre la couche basale et la couche cornée, par une méthode selon l'une des revendications 4 à 7, et
- sa comparaison au différentiel de différenciation épidermique moyen existant au sein d'un épiderme normal de même type, entre la couche basale et la couche cornée.

12. Utilisation du dosage de l'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c, dans des kératinocytes au sein d'un échantillon d'épithélium humain isolé pour la détermination de l'état de différenciation épidermique desdits kératinocytes.

13. Utilisation in vitro d'un kit pour la détermination de l'état de différenciation d'un kératinocyte humain, comprenant des moyens pour le dosage de l'expression des microARNs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a et hsa-miR-30c et la mention d'un niveau de référence pour l'expression desdits microARNs.

## Patentansprüche

1. Methode zur Bestimmung des epidermalen Differenzierungszustands eines menschlichen Keratinozyten in einer Probe isolierten menschlichen Epithels, umfassend
- Bestimmen des Expressionsniveaus der microRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a und hsa-miR-30c in dem Keratinozyt und
- Vergleichen des Expressionsniveaus der microRNAs mit dem durchschnittlichen Expressionsniveau dieser microRNAs in gezüchteten nicht differenzierten Keratinozyten oder in differenzierten epidermalen Keratinozyten, die vorzugsweise aus demselben Stamm oder derselben Population stammen.

2. Methode nach Anspruch 1,
wobei der das Keratinozyt als differenziert angesehen wird, wenn das Expressionsniveau der microRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 und hsa-miR-92b statistisch über dem durchschnittlichen Expressionsniveau der entsprechenden microRNAs in gezüchteten nicht differenzierten Keratinozyten liegt.

3. Methode nach Anspruch,
wobei der das Keratinozyt als nicht differenziert angesehen wird, wenn das Expressionsniveau der microRNAs hsa-let-7i, hsa-miR-22, hsa-miR-221, hsa-miR-222, hsa-miR-29a, hsa-miR-29b, hsa-miR-663, hsa-miR-30a und hsa-miR-30c statistisch über dem durchschnittlichen Expressionsniveau der entsprechenden microRNAs in differenzierten epidermalen Keratinozyten liegt.

4. Methode zur Bewertung des in einer Probe isolierten menschlichen Epithels bestehenden epidermalen Differenzierungsunterschieds, umfassend den Vergleich des Expressionsniveaus der microRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a und hsa-miR-30c zwischen zwei Keratinozytenstämmen dieses Epithels.

5. Methode nach Anspruch 4,
wobei der der Unterschied der epidermalen Differenzierung zwischen den beiden Stämmen sich durch eine stärkere Expression der microRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 und hsa-miR-92b in einem Stamm in Bezug auf den anderen mit einem Faktor größer 1,3, vorzugsweise größer 1,5 auszeichnet.

6. Methode nach Anspruch 4,
wobei der der Unterschied der epidermalen Differenzierung zwischen den beiden Stämmen sich durch eine stärkere Expression der microRNAs hsa-let-7i, hsa-miR-22, hsa-miR-221, hsa-miR-222, hsa-miR-29a, hsa-miR-29b, hsa-miR-663, hsa-miR-30a und hsa-miR-30c in einem Stamm in Bezug auf den anderen mit einen Faktor größer 1,3, vorzugsweise größer 1,5 auszeichnet.

7. Methode nach einem der Ansprüche 1 bis 6,
wobei der die Probe von einer menschlichen Haut oder einem Kulturepithel vom Typ "wiederhergestellte Haut" stammt.

8. Verfahren zum Bestimmen der Wirksamkeit einer Behandlung eines Epithels, umfassend den Vergleich des Expressionsniveaus der microRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a und hsa-miR-30c in Keratinozyten dieses Epithels vor und nach der Behandlung, wobei das Verfahren nicht an Menschen durchgeführt wird.

9. Verfahren nach Anspruch 8,
wobei dem die Behandlung als wirksam angesehen wird, wenn sie eine Veränderung des Expressionsniveaus von wenigstens einer, vorzugsweise wenigstens zwei oder fünf der microRNAs innerhalb des Epithels bewirkt.

10. Verfahren zum In-vitro- oder Ex-vivo-Screening von Modulatoren des epidermalen Differenzierungsprozesses innerhalb eines Epithels, umfassend die Bewertung des epidermalen Differenzierungszustands von Keratinozyten in diesem Epithel durch eine Methode nach einem der Ansprüche 1 bis 3 vor und nach Anwendung des zu screenenden Modulators.

11. Verfahren zur Diagnose des Zustands einer Epidermis, umfassend:
- Bestimmen des in der Epidermis zwischen der Basalschicht und der Hornschicht bestehenden epidermalen Differenzierungsunterschieds durch eine Methode nach einem der Ansprüche 4 bis 7, und
- Vergleichen von diesem mit dem in einer normalen Epidermis gleichen Typs zwischen der Basalschicht und der Hornschicht bestehenden durchschnittlichen epidermalen Differenzierungsunterschied.

12. Verwendung der Expressionsanalyse der microRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a und hsa-miR-30c in Keratinozyten in einer Probe isolierten menschlichen Epithels zur Bestimmung des epidermalen Differenzierungszustands der Keratinozyten.

13. In-vitro-Verwendung eines Kits zur Bestimmung des Differenzierungszustands eines menschlichen Keratinozyten, umfassend Mittel zur Expressionsanalyse der microRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a und hsa-miR-30c und Angabe eines Referenzniveaus für die Expression dieser microRNAs.

## Claims

1. Method for determining the state of epidermal differentiation of a human keratinocyte in a sample of isolated human epithelium, comprising:
• determining the level of expression of the miRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a and hsa-miR-30c, within said keratinocyte; and
• comparing the level of expression of the miRNAs with the mean level of expression of these miRNAs in non-differentiated keratinocytes under culture, or in differentiated epidermal keratinocytes, preferably originating from the same strain or the same population.

2. The method according to claim 1, wherein said keratinocyte is considered to be differentiated if the level of expression of the miRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 and hsa-miR-92b is statistically greater than the mean level of expression of said miRNAs in non-differentiated keratinocytes under culture.

3. The method according to claim 1, wherein said keratinocyte is considered to be non-differentiated if the level of expression of the miRNAs hsa-let-7i, hsa-miR-22, hsa-miR-221, hsa-miR-222, hsa-miR-29a, hsa-miR-29b, hsa-miR-663, hsa-miR-30a and hsa-miR-30c is statistically greater than the mean level of expression of said miRNAs in differentiated epidermal keratinocytes.

4. A method for evaluating the differential in epidermal differentiation existing in a sample of isolated human epithelium, comprising comparing, between two layers of keratinocytes of said epithelium, the level of expression of the miRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a and hsa-miR-30c.

5. The method according to claim 4, wherein the differential in epidermal differentiation between the two layers is **characterized by** greater expression of the miRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425 and hsa-miR-92b in one layer compared with another, by a factor greater than 1.3, preferably greater than 1.5.

6. The method according to claim 4, wherein the differential in epidermal differentiation between the two layers is **characterized by** greater expression of the miRNAs hsa-let-7i, hsa-miR-22, hsa-miR-221, hsa-miR-222, hsa-miR-29a, hsa-miR-29b, hsa-miR-663, hsa-miR-30a and hsa-miR-30c in one layer compared with another, by a factor greater than 1.3, preferably greater than 1.5.

7. The method according to any one of claims 1 to 6, wherein said sample originates from human skin or from a "reconstructed skin" type cultured epithelium.

8. A method for determining the efficacy of a treatment of an epithelium, comprising comparing, before and after treatment, the level of expression of the miRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a and hsa-miR-30c within keratinocytes of said epithelium, wherein said method is not practised on human beings.

9. The method according to claim 8, wherein said treatment is considered to be effective if it causes a modification in the level of expression of at least one, preferably at least two or five of said miRNAs in the epithelium.

10. A method for screening *in vitro* or *ex vivo* modulators of the epidermal differentiation process in an epithelium, comprising evaluating the state of epidermal differentiation of keratinocytes in said epithelium using a method according to any one of claims 1 to 3, before and after application of the modulator to be screened.

11. A method for diagnosing the state of an epidermis, comprising:
• determining the differential in epidermal differentiation existing within said epidermis between the basal layer and the stratum corneum, using a method according to any one of claims 4 to 7; and
• comparing it with the mean differential in epidermal differentiation existing in a normal epidermis of the same type, between the basal layer and the stratum corneum.

12. Use of an assay of the expression of the miRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a and hsa-miR-30c, in keratinocytes in a sample of isolated human epithelium, to determine the state of epidermal differentiation of said keratinocytes.

13. *In vitro* use of a kit for determining the state of differentiation of a human keratinocyte, comprising means for assaying the expression of the miRNAs hsa-miR-455-3p, hsa-miR-141, hsa-miR-148a, hsa-miR-182, hsa-miR-224, hsa-miR-26a, hsa-miR-26b, hsa-miR-361-5p, hsa-miR-425, hsa-miR-92b, hsa-let-7i, hsa-miR-22, hsa-miR-221, hsa-miR-222, hsa-miR-29a, hsa-miR-29b, hsa-miR-663, hsa-miR-30a and hsa-miR-30c and information providing a reference level for expression of said miRNAs.


### SEQUENCE LISTING

<110> L'OREAL
<120> signature microARN de la différenciation épidermique et utilisations
<130> B08358A CS
<150> US 61/239154
   <151> 2009-09-02
<150> FR 09/05789
   <151> 2009-12-01
<160> 19
<170> PatentIn version 3.3
<210> 1
   <211> 22
   <212> RNA
   <213> homo sapiens
<400> 1
   uaacacuguc ugguaaagau gg 22
<210> 2
   <211> 22
   <212> RNA
   <213> homo sapiens
<400> 2
   ucagugcacu acagaacuuu gu 22
<210> 3
   <211> 24
   <212> RNA
   <213> homo sapiens
<400> 3
   uuuggcaaug guagaacuca cacu 24
<210> 4
   <211> 21
   <212> RNA
   <213> homo sapiens
<400> 4
   caagucacua gugguuccgu u 21
<210> 5
   <211> 22
   <212> RNA
   <213> homo sapiens
<400> 5
   uucaaguaau ccaggauagg cu 22
<210> 6
   <211> 21
   <212> RNA
   <213> homo sapiens
<400> 6
   uucaaguaau ucaggauagg u 21
<210> 7
   <211> 22
   <212> RNA
   <213> homo sapiens
<400> 7
   uuaucagaau cuccaggggu ac 22
<210> 8
   <211> 23
   <212> RNA
   <213> homo sapiens
<400> 8
   aaugacacga ucacucccgu uga 23
<210> 9
   <211> 21
   <212> RNA
   <213> homo sapiens
<400> 9
   gcaguccaug ggcauauaca c 21
<210> 10
   <211> 22
   <212> RNA
   <213> homo sapiens
<400> 10
   uauugcacuc gucccggccu cc 22
<210> 11
   <211> 22
   <212> RNA
   <213> homo sapiens
<400> 11
   ugagguagua guuugugcug uu 22
<210> 12
   <211> 22
   <212> RNA
   <213> homo sapiens
<400> 12
   aagcugccag uugaagaacu gu 22
<210> 13
   <211> 23
   <212> RNA
   <213> homo sapiens
<400> 13
   agcuacauug ucugcugggu uuc 23
<210> 14
   <211> 21
   <212> RNA
   <213> homo sapiens
<400> 14
   agcuacaucu ggcuacuggg u 21
<210> 15
   <211> 22
   <212> RNA
   <213> homo sapiens
<400> 15
   uagcaccauc ugaaaucggu ua 22
<210> 16
   <211> 23
   <212> RNA
   <213> homo sapiens
<400> 16
   uagcaccauu ugaaaucagu guu 23
<210> 17
   <211> 22
   <212> RNA
   <213> homo sapiens
<400> 17
   aggcggggcg ccgcgggacc gc 22
<210> 18
   <211> 22
   <212> RNA
   <213> homo sapiens
<400> 18
   uguaaacauc cucgacugga ag 22
<210> 19
   <211> 23
   <212> RNA
   <213> homo sapiens
<400> 19
   uguaaacauc cuacacucuc agc 23